# EUROPEAN PATENT SPECIFICATION

(11) **EP 3 011 916 B1**
(45) Date of publication and mention of the grant of the patent: **20.09.2017**
(21) Application number: 15191313.4
(22) Date of filing: 23.10.2015
(51) Int. Cl.: A61B 17/128, A61B 17/00

(54) **REPOSABLE MULTIPLATFORM ENDOSCOPIC SURGICAL CLIP APPLIER**
AUSWECHSELBARE ENDOSKOPISCHE CHIRURGISCHE MEHRFACHPLATTFORMKLAMMER
APPLICATEUR D'AGRAFES CHIRURGICALES ENDOSCOPIQUES MULTI-PLATEFORME RÉUTILISABLE

(30) Priority: 24.10.2014 US 201462068341 P; 06.10.2015 US 201514876060
(43) Date of publication of application: 27.04.2016
(73) Proprietor: Covidien LP, Mansfield, MA 02048 (US)
(72) Inventor: HOLSTEN, Henry, Hamden, CT 06518 (US)
(74) Representative: Soames, Candida Jane

(56) References cited:
- EP-A1- 0 622 049
- EP-A2- 0 755 655
- US-A- 6 099 537

## Description

### BACKGROUND

### Technical Field

The technical field relates to surgical clip appliers. More particularly, the present disclosure relates to a reposable multiplatform endoscopic surgical clip applier having a handle assembly configured for use with a plurality of different adapter assemblies and/or clip cartridge assemblies for firing different types and/or sizes of clips.

### Description of Related Art

Endoscopic staplers and clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures. Typically, a tube or cannula device is extended into the patient's body through the entrance incision to provide an access port. The port allows the surgeon to insert a number of different surgical instruments therethrough for performing surgical procedures far removed from the incision.

During a majority of these procedures, the surgeon must often terminate the flow of blood or another fluid through one or more vessels. The surgeon will often apply a surgical clip to a blood vessel or another duct to prevent the flow of body fluids therethrough during the procedure. An endoscopic clip applier is known in the art for applying a single clip during an entry to the body cavity. Such clips are typically fabricated from a biocompatible material and are usually compressed over a vessel. Once applied to the vessel, the compressed clip terminates the flow of fluid therethrough.

Endoscopic clip appliers that are able to apply multiple clips in endoscopic or laparoscopic procedures during a single entry into the body cavity are described in commonly-assigned U.S. Patent Nos. 5,084,057 and 5,100,420 to Green et al. Another multiple endoscopic clip applier is disclosed in commonly-assigned U.S. Patent No. 5,607,436 by Pratt et al. These devices are typically, though not necessarily, used during a single surgical procedure. U.S. Patent Application No. 08/515,341 now U.S. Patent No. 5,695, 502 to Pier et al., discloses a resterilizable surgical clip applier. This resterilizable clip applier is configured to receive and cooperate with an interchangeable clip magazine so as to advance and form multiple clips during a single entry into a body cavity. EP0755655 discloses a clip applier according to the preamble of claim 1.

During endoscopic or laparoscopic procedures it may be desirable and/or necessary to use different size surgical clips depending on the underlying tissue or vessels to be ligated. In order to reduce overall costs of a surgical clip applier, it is desirable for a single surgical clip applier to be loadable with and capable of firing different size surgical clips as needed.

Accordingly, a need exists for endoscopic surgical clip appliers that include handle assemblies configured for use with different adapter assemblies and/or clip cartridge assemblies for firing different types and/or sizes of clips.

### SUMMARY

A reposable endoscopic surgical clip applier according to the invention is recited in claim 1. A method of assembling said clip applier according to the invention is recited in claim 9. Preferred embodiments of the invention are recited in the dependent claims. Reposable endoscopic surgical clip appliers including handle assemblies configured for use with different adapter assemblies and/or clip cartridge assemblies for firing different types and/or sizes of clips are provided in accordance with the present disclosure. To the extent consistent, any of the aspects or features of the present disclosure may be used in conjunction with any of the other aspects and features of the present disclosure.

A handle assembly for a reposable endoscopic surgical clip applier provided in accordance with the present disclosure includes a housing, a trigger, a plunger assembly, and a plug. The housing includes a stationary handle and a barrel supported on the stationary handle. The barrel includes a proximal end, a distal end, and a longitudinal axis. The barrel further defines a lumen extending between the proximal and distal ends thereof. The lumen is aligned on the longitudinal axis and includes a proximal portion and a distal portion. The distal portion of the lumen is configured to releasably receive an adapter assembly operably associated with a clip cartridge assembly, an end effector, and/or a surgical tool. The trigger is operably coupled to the housing and includes a gripping portion and a stem portion. The gripping portion is movable relative to the stationary handle from an un-actuated position to an actuated position to move the stem portion distally through the proximal portion of the lumen. The plunger assembly is removably positionable within the proximal portion of the lumen and is configured to operably couple the stem portion with an adapter assembly received within the distal portion of the lumen such that distal movement of the stem portion through the proximal portion of the lumen actuates the adapter assembly. The plug is removably engagable within the proximal end of the barrel to close off the proximal portion of the lumen, thereby retaining the stem portion, the plunger assembly, and the adapter assembly in operable relation relative to one another.

In aspects of the present disclosure, the plunger assembly includes a plunger having a head and a shaft extending distally from the head. In such aspects, upon distal movement of the stem portion through the proximal portion of the lumen, the stem portion is urged into contact with the head to thereby urge the plunger distally to actuate the adapter assembly.

In aspects of the present disclosure, the plunger assembly further includes a flange slidably disposed about the shaft and a biasing member disposed about the shaft and positioned between the flange and the head. In such aspects, the flange may be configured to abut a shoulder defined within the proximal portion of the lumen when the plunger assembly is positioned therein such that the biasing member is compressed upon distal movement of the plunger through the proximal portion of the lumen.

In aspects of the present disclosure, the plug is configured for threaded engagement within the proximal end of the barrel.

In aspects of the present disclosure, a lock collar is provided. The lock collar is rotatably coupled to the barrel and extends distally therefrom. The lock collar is rotatable relative to both the barrel and the adapter assembly to releasably engage the adapter assembly within the distal portion of the lumen.

In aspects of the present disclosure, the housing, the trigger, the plunger assembly, and the plug are configured as resterilizable, reusable components.

A reposable endoscopic surgical clip applier provided in accordance with aspects of the present disclosure includes a handle assembly and a plurality of adapter assemblies. The handle assembly includes a housing, a trigger, a plunger assembly, and a plug. The housing includes a stationary handle and a barrel supported on the stationary handle. The barrel includes a proximal end, a distal end, and a longitudinal axis. The barrel defines a lumen extending between the proximal and distal ends thereof. The lumen is aligned on the longitudinal axis and includes a proximal portion and a distal portion. The trigger is operably coupled to the housing and movable relative to the stationary handle from an un-actuated position to an actuated position. The plunger assembly is removably positionable within the proximal portion of the lumen and is configured to operably couple to the trigger such that movement of the trigger from the un-actuated position to the actuated position advances the plunger assembly distally though the proximal portion of the lumen. The plug is removably engagable within the proximal end of the barrel to close off the proximal portion of the lumen, thereby retaining the trigger in operable relation relative to the plunger assembly.

Each of the adapter assemblies is operably associated with a clip cartridge assembly, an end effector, and/or a surgical tool and is configured for releasable receipt with the distal portion of the lumen in operable relation relative to the plunger assembly such that distal movement of the plunger assembly through the proximal portion of the lumen actuates the clip cartridge assembly, end effector, or surgical tool associated with that adapter assembly. At least one adapter assembly of the plurality of adapter assemblies has a stroke length which is different from a stroke length of the remaining adapter assemblies.

In aspects of the present disclosure, the trigger includes a gripping portion and a stem portion. The gripping portion of the trigger is movable relative to the stationary handle from the un-actuated position to the actuated position to move the stem portion distally through the proximal portion of the lumen to advance the plunger assembly distally though the proximal portion of the lumen.

In aspects of the present disclosure, the plunger assembly includes a plunger having a head and a shaft extending distally from the head. Upon distal movement of the stem portion through the proximal portion of the lumen, the stem portion is urged into contact with the head to thereby urge the plunger distally.

In aspects of the present disclosure, the plunger assembly further includes a flange slidably disposed about the shaft and a biasing member disposed about the shaft and positioned between the flange and the head. In such aspects, the flange may be configured to abut a shoulder defined within the proximal portion of the lumen when the plunger assembly is positioned therein such that the biasing member is compressed upon distal movement of the plunger through the proximal portion of the lumen.

In aspects of the present disclosure, the handle assembly further includes a lock collar rotatably coupled to the barrel and extending distally therefrom. With one of the adapter assemblies inserted into the distal portion of the lumen, the lock collar is rotatable relative to the barrel and that adapter assembly to releasably engage that adapter assembly within the distal portion of the lumen.

In aspects of the present disclosure, one or more of the adapter assemblies includes an outer shell, a collar slidably disposed within the outer shell, a shaft engaged with the collar and extending distally from the collar and the outer shell, and a plunger assembly slidably disposed within the outer shell. In such aspects, the plunger assembly of the adapter assembly may include a stem and a plunger slidable relative to the stem and including a head and a shaft extending distally from the head. Further still, in such aspects, the plunger assembly may include a biasing member disposed about the stem and the shaft of the plunger and positioned between the collar and the head of the plunger such that slidable insertion of the shaft of the plunger into the stem compresses the biasing member.

In aspects of the present disclosure, distal movement of the plunger of the handle assembly urges the plunger of the adapter assembly distally to actuate the clip cartridge assembly, end effector, or surgical tool associated with that adapter assembly.

A method of assembling a reposable endoscopic surgical clip applier provided in accordance with aspects of the present disclosure includes inserting a plunger assembly into a proximal portion of a lumen defined within a barrel of a housing. The housing further includes a stationary handle supporting the barrel thereon. The method further includes engaging a plug within the proximal portion of the lumen of the barrel to close off the proximal portion of the lumen of the barrel and retain the plunger assembly therein, inserting an adapter assembly into a distal portion of the lumen of the barrel such that the adapter assembly is operably positioned relative to the plunger assembly, and engaging the adapter assembly within the distal portion of the lumen of the barrel.

In aspects of the present disclosure, the method further includes, prior to engaging the plug, moving a gripping portion of a trigger operably associated with the housing towards the stationary handle such that a stem portion of the trigger abuts a proximal end of the plunger assembly.

In aspects of the present disclosure, engaging the adapter assembly within the distal portion of the lumen of the barrel includes rotating a lock collar relative to both the adapter assembly and the barrel.

### BRIEF DESCRIPTION OF THE DRAWINGS

Aspects and features of the present disclosure are described herein with reference to the accompanying drawings, wherein:
FIG. 1 is an exploded, perspective view of a handle assembly of a reposable endoscopic surgical clip applier provided in accordance with aspects of the present disclosure;
FIG. 2 is a partial longitudinal, cross-sectional view of the handle assembly of FIG. 1, with a first adapter assembly operably engaged therein;
FIG. 3 is a perspective view of a proximal end of the first adapter assembly of FIG. 2 and a proximal end of a second adapter assembly provided in accordance with aspects of the present disclosure and configured for use with the handle assembly of FIG. 1;
FIG. 4 is a perspective view of the proximal end of the first adapter assembly of FIG. 2 with the outer tubular shell removed to illustrate the internal components thereof;
FIG. 5 is a perspective view of the proximal end of the first adapter assembly of FIG. 2 with a portion of the outer tubular shell removed to illustrate the internal components thereof; and
FIG. 6 is a perspective view of the second adapter assembly of FIG. 3 illustrating the tubular shaft thereof extending distally from the outer tubular shell thereof.

### DETAILED DESCRIPTION OF EMBODIMENTS

Embodiments of reposable endoscopic surgical clip appliers provided in accordance with the present disclosure will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus or component thereof which is closer to the user and the term "distal" refers to the end of the apparatus or component thereof which is further from the user.

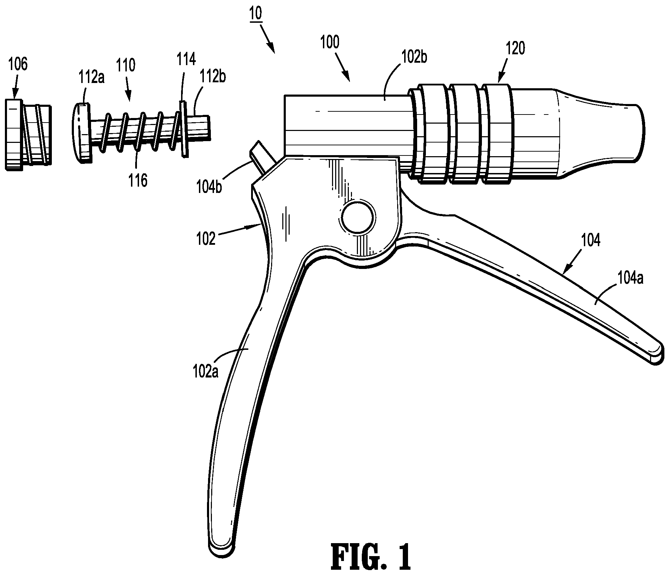

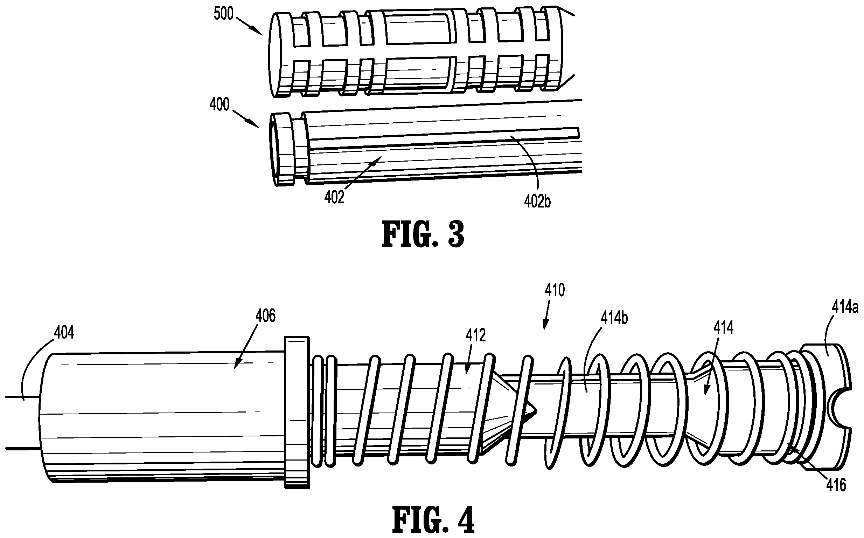

Turning to FIGS. 1-3, an endoscopic surgical clip applier provided in accordance with the present disclosure is generally designated as 10. Surgical clip applier 10 generally includes a handle assembly 100 and a plurality of adapter assemblies, e.g., adapter assemblies 400, 500 (FIG. 3), configured for releasable engagement and use with handle assembly 100. Each adapter assembly 400, 500 is configured for actuating one or more particular clip cartridge assemblies (not shown), end effectors (not shown), or surgical tools (not shown). The adapter assemblies 400, 500 may be reusable components configured for releasable engagement and use with one or more disposable clip cartridge assemblies, disposable/reusable end effectors, and/or disposable/reusable surgical tools. Alternatively, the adapter assemblies 400, 500 may be integral with corresponding clip cartridge assemblies, end effectors, or surgical tools and, together, may be reusable or disposable. Handle assembly 100 is configured as a reusable component.

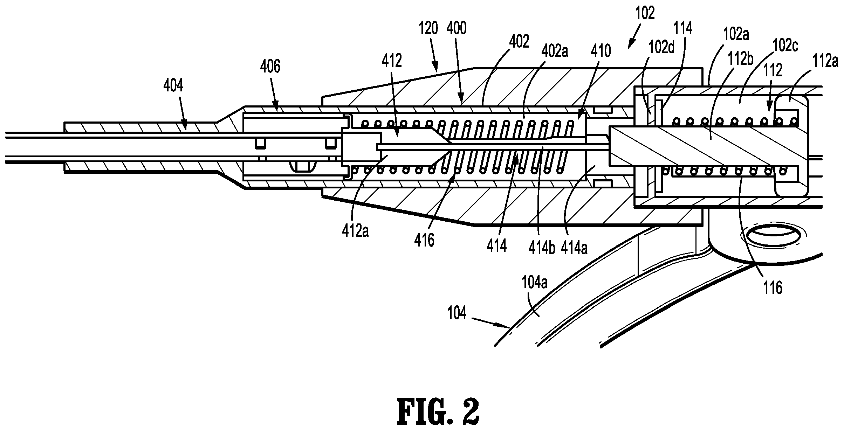

With reference to FIG. 1, handle assembly 100 of surgical clip applier 10 includes a housing 102, a trigger 104, a plug 106, and a plunger assembly 110. Housing 102 includes a stationary handle portion 102a, and a barrel portion 102b supported on stationary handle portion 102a. Barrel portion 102b defines a lumen 102c (FIG. 2) therethrough. In some embodiments, barrel portion 102b includes a lock collar 120 rotatably coupled to barrel portion 102b and extending distally therefrom. Housing 102 of handle assembly 100 may be formed of a suitable plastic or thermoplastic material, or from stainless steel or the like.

Trigger 104 of handle assembly 100 is pivotably connected to stationary handle portion 102a of housing 102. Trigger 104 includes a finger grip portion 104a projecting from stationary handle portion 102a and/or barrel portion 102b of housing 102, and an actuation stem 104b configured to move into lumen 102c (FIG. 2) of barrel portion 102b of housing 102, as detailed below. Trigger 104 may be formed of a suitable plastic or thermoplastic material, or from stainless steel or the like.

Plug 106 of handle assembly 100 is releasably securable within a proximal end of barrel portion 102b of housing 102 for closing the proximal end of barrel portion 102b and lumen 102c (FIG. 2) of housing 102. Plug 106 may be connectable to barrel portion 102b of housing 102 via screw threads, a bayonet-type arrangement, or other suitable releasable engagement structure. Plug 106 may be formed of a suitable plastic or thermoplastic material, or from stainless steel or the like.

Plunger assembly 110 of handle assembly 100 is removably supported within lumen 102c (FIG. 2) of barrel portion 102b of housing 102. Plunger assembly 110 includes a plunger 112 having a head portion 112a and a shaft portion 112b extending distally from head portion 112a. Plunger assembly 110 further includes a flange 114 slidably supported on shaft portion 112b at a location proximal of a distal tip of shaft portion 112b. The distal tip of shaft portion 112b may have an enlarged radial profile, or other suitable feature, thereby preventing flange 114 from being removed from the distal end of shaft portion 112b. Head portion 112a of plunger 112 inhibits flange 114 from being removed from the proximal end of shaft portion 112b. A biasing member 116 of plunger assembly 110 is disposed about shaft portion 112b and interposed between head portion 112a of plunger 112 and flange 114 to bias flange 114 distally relative to shaft portion 112b. The components of plunger assembly 110 may be formed of a suitable plastic or thermoplastic material, or from stainless steel or the like.

With additional reference to FIG. 2, when plunger assembly 110 is loaded into lumen 102c of barrel portion 102b of housing 102, the distal tip of shaft portion 112b of plunger 112 slidably extends through an aperture in a support wall 102d of housing 102, and flange 114 rests adjacent to or in contact with a proximal surface of support wall 102d of housing 102. Following loading of plunger assembly 110 into lumen 102c of barrel portion 102b of housing 102, trigger 104 is pivoted until actuation stem 104b of trigger 104 is moved into lumen 102c of barrel portion 102b adjacent to or in contact with head portion 112a of plunger 112. Plug 106 may then be connected to barrel portion 102b of housing 102 to maintain actuation stem 104b of trigger 104 adjacent to or in operative contact with head portion 112a of plunger 112. At this point, actuation stem 104b is inhibited from returning proximally and is only permitted to move distally upon actuation of trigger 104 towards stationary handle 102a of housing 102.

Turning to FIGS. 2-6, as mentioned above, surgical clip applier 10 may include a plurality of adapter assemblies, e.g., a first adapter assembly 400 and a second adapter assembly 500, each configured for operable coupling with handle assembly 100 and interfacing with one or more specific clip cartridge assemblies, end effectors, and/or surgical tools.

With particular reference to FIGS. 2, 4, and 5, first adapter assembly 400 includes an outer tubular shell 402, a tubular shaft 404, and a plunger assembly 410. Shell 402 defines a lumen 402a therethrough. Shell 402 is configured and dimensioned for selective receipt within and connection to a distal portion of lumen 102c of barrel portion 102b of housing 102. Lumen 402a of shell 402 includes a relatively larger diameter proximal portion, and a relatively smaller diameter distal portion.

Shaft 404 of first adapter assembly 400 is fixedly secured to and extends distally from shell 402. Shaft 404 includes a proximal end disposed within the relatively smaller diameter distal portion of shell 402 and extending at least partially into the relatively larger diameter proximal portion of shell 402. A collar 406 is disposed within the relatively larger diameter proximal portion of shell 402 to which the proximal end of shaft 404 is fixedly secured.

Plunger assembly 410 of first adapter assembly 400 is disposed within the relatively larger diameter proximal portion of shell 402. Plunger assembly 410 includes a stem 412 disposed proximally of collar 406. Stem 412 defines a lumen 412a therethrough which is axially aligned with shaft 404. Plunger assembly 410 further includes a plunger 414 having a head portion 414a and a shaft portion 414b extending from head portion 414a. Shaft portion 414b extends into lumen 412a of stem 412 and is slidable relative thereto. Plunger assembly 410 also includes a biasing member 416 disposed about stem 412 of shaft portion 414b of plunger 414 and interposed between head portion 414a of plunger 414 and collar 406 so as to bias plunger 414 proximally relative to collar 406.

First adapter assembly 400 may be connected to handle assembly 100 by a bayonet-type arrangement by inserting shell 402 into the distal portion of lumen 102c of barrel portion 102b of housing 102, and then rotating shell 402 of first adapter assembly 400 relative to barrel portion 102b of housing 102 to engage the thread(s) within the corresponding groove(s) of the bayonet coupling (not shown). Alternatively, first adapter assembly 400 may be connected to handle assembly 100 by inserting shell 402 into the distal portion of lumen or passage 102c of barrel portion 102b of housing 102, and then rotating lock collar 120 of handle assembly 100 relative to both housing 102 and shell 402 to engage the thread(s) with the corresponding groove(s) of the bayonet coupling. Other suitable releasable engagement structures, e.g., mechanical latches, snap-fit connections, etc., are also contemplated.

As best shown in FIG. 3, shell 402 of first adapter assembly 400 may be provided with a longitudinally extending rib 402b. Rib 402b of shell 402 acts like a clocking feature to radially orient and align first adapter assembly 400 with handle assembly 100 for proper connection or coupling of first adapter assembly 400 and handle assembly 100. Handle assembly 100 may include a complementary recess (not shown) or other suitable feature configured to receive rib 402b and align first adapter assembly 400 relative to handle assembly 100. In embodiments where rib 402b is provided, lock collar 120 may also be provided such that lock collar 120 may be rotated relative to both housing 102 and shell 402 (while housing 102 and shell 402 remain stationary relative to one another) to engage first adapter assembly 400 with handle assembly 100, as noted above, thereby maintaining the proper orientation of first adapter assembly 400 relative to handle assembly 100.

Referring to FIGS. 1-5, in operation, with first adapter assembly 400 coupled with handle assembly 100 as detailed above, with plunger assembly 110 loaded in housing 102 as also detailed above, and with a clip cartridge assembly, end effector, or surgical tool connected or coupled to first adapter assembly 400, surgical clip applier 10 is ready for use. In use, as trigger 104 of handle assembly 100 is actuated towards stationary handle portion 102b of housing 102, stem 104b of trigger 104 engages head portion 112a of plunger 112 of plunger assembly 110 and distally advances plunger 112, thereby compressing biasing member 116 between flange 114 and head portion 112a of plunger 112 of plunger assembly 110. As plunger 112 is advanced distally, shaft portion 112b of plunger 112 engages and acts on head portion 414a of plunger assembly 410 of first adapter assembly 400.

With shaft portion 112b of plunger assembly 112 engaging and acting on head portion 414a of plunger 414 of plunger assembly 410 of first adapter assembly 400, plunger 414 is advanced distally, thereby compressing biasing member 416 between collar 406 and head portion 414a of plunger 414. As plunger 414 is advanced in a distal direction, shaft portion 414b of plunger 414 is advanced in a distal direction through lumen 412a of stem 412.

Upon sufficient distal advancement of shaft portion 414b of plunger 414, a distal end of shaft portion 414b of plunger 414 is sufficiently advanced through or beyond lumen 412a of stem 412 so as to act on or otherwise engage the clip cartridge assembly, end effector, or surgical tool operatively connected to first adapter assembly 400 to actuate the respective clip cartridge assembly, end effector, or surgical tool.

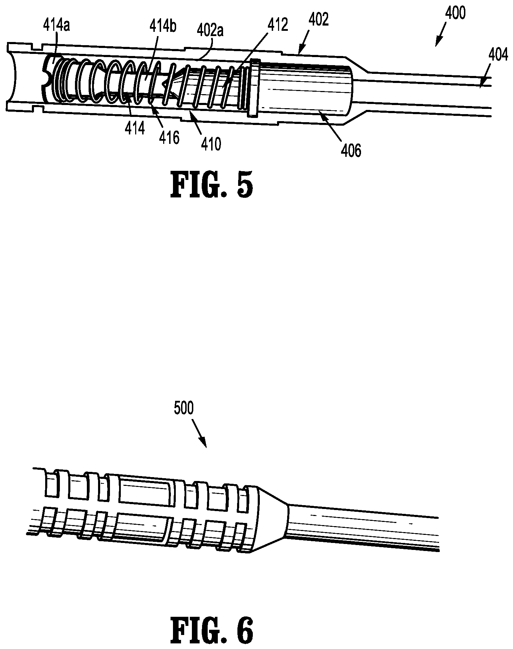

Referring to FIGS. 3 and 6, in accordance with the present disclosure, first adapter assembly 400 may be replaced by second adapter assembly 500, or any other suitable adapter assembly having a particular clip cartridge assembly, end effector, or surgical tool operably associated therewith. By replacing first adapter assembly 400 with, for example, second adapter assembly 500, handle assembly 100 (FIG. 1) is capable of actuating the corresponding clip cartridge assembly, end effector, or surgical tool operably associated with second adapter assembly 500, e.g., to actuate different types and/or sizes of clips.

In accordance with the present disclosure, each adapter assembly, for example, adapter assemblies 400 and 500, may have a different or unique stroke length from one another in order to accommodate a particular function of the adapter assembly 400 or 500.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

## Claims

1. A reposable endoscopic surgical clip applier (10), comprising:
a handle assembly (100), including:
a housing (102) including a stationary handle (102a) and a barrel (102b) supported on the stationary handle, the barrel including a proximal end, a distal end, and a longitudinal axis, the barrel defining a lumen (102c) extending between the proximal and distal ends thereof, the lumen aligned on the longitudinal axis and including a proximal portion and a distal portion;
a trigger (104) operably coupled to the housing (102) and movable relative to the stationary handle (102a) from an un-actuated position to an actuated position;
a plunger assembly (110) removably positionable within the proximal portion of the lumen, the plunger assembly including a plunger (112) having a head (112a) and a shaft (112b) extending distally from the head and being configured to operably couple to the trigger such that movement of the trigger from the un-actuated position to the actuated position advances the plunger assembly distally though the proximal portion of the lumen; and
a plug (106) removably engagable within the proximal end of the barrel (102b) to close off the proximal portion of the lumen (102c), thereby retaining the trigger in operable relation relative to the plunger assembly (100); the clip applier further comprising
a plurality of adapter assemblies (400, 500), each of which is operably associated with at least one of: a clip cartridge assembly, an end effector, or a surgical tool, **characterized in that** at least one of the adapter assemblies includes:
an outer shell (402);
a collar (406) slidably disposed within the outer shell;
a shaft (404) engaged with the collar and extending distally from the collar and the outer shell; and
a plunger assembly (410) slidably disposed within the outer shell, the plunger assembly including a stem (412) and a plunger (414) slidable relative to the stem, the plunger including a head (414a) and a shaft (414b) extending distally from the head, wherein each adapter assembly is configured for releasable receipt with the distal portion of the lumen (102c) in operable relation relative to the plunger assembly (110) of the handle assembly (100) such that distal movement of the plunger (112) of the handle assembly urges the plunger (414) of the at least one adapter assembly distally to actuate the clip cartridge assembly, end effector, or surgical tool associated with that adapter assembly.

2. The clip applier according to claim 1, wherein the plurality of adapter assemblies (400, 500) has a stroke length which is different from a stroke length of the remaining adapter assemblies.

3. The clip applier according to claim 1 or 2, wherein the trigger (104) includes a gripping portion (104a) and a stem portion (104b), the gripping portion movable relative to the stationary handle (102a) from the un-actuated position to the actuated position to move the stem portion distally through the proximal portion of the lumen to advance the plunger assembly of the handle assembly distally though the proximal portion of the lumen.

4. The clip applier according to claim 1, 2 or 3, wherein upon distal movement of the stem portion(104b) through the proximal portion of the lumen (102c), the stem portion is urged into contact with the head (112a) to thereby urge the plunger (112) distally; preferably wherein the plunger assembly (110) further includes a flange (114) slidably disposed about the shaft (112b) and a biasing member (116) disposed about the shaft and positioned between the flange and the head; preferably still wherein the flange is configured to abut a shoulder defined within the proximal portion of the lumen when the plunger assembly is positioned therein such that the biasing member is compressed upon distal movement of the plunger through the proximal portion of the lumen.

5. The clip applier according to any one of the preceding claims, wherein the handle assembly (100) further includes a lock collar (120) rotatably coupled to the barrel (102b) and extending distally therefrom, and wherein, with one of the adapter assemblies inserted into the distal portion of the lumen (102c), the lock collar is rotatable relative to the barrel and that adapter assembly to releasably engage that adapter assembly within the distal portion of the lumen.

6. The clip applier according to any one of the preceding claims, wherein the plunger assembly (410) of the at least one adapter assembly further includes a biasing member (416) disposed about the stem (412) and the shaft (414b) of the plunger (414) and positioned between the collar (406) and the head (414a) of the plunger, the plunger assembly configured such that slidable insertion of the shaft of the plunger into the stem compresses the biasing member.

7. The clip applier according to any preceding claim, wherein the plug (106) of the handle assembly (100) is configured for threaded engagement within the proximal end of the barrel (102b).

8. The clip applier according to any preceding claim, wherein the housing (102), the trigger (104), the plunger assembly (110), and the plug (106) are configured as resterilizable, reusable components.

9. A method of assembling a reposable endoscopic surgical clip applier (10) according to any one of the preceding claims, comprising:
inserting the plunger assembly (110) of the handle assembly (100) into a proximal portion of the lumen (102c) defined within the barrel (102b) of the housing (102);
engaging the plug (106) within the proximal portion of the lumen (102c) of the barrel to close off the proximal portion of the lumen of the barrel and retain the plunger assembly (110) therein;
inserting an adapter assembly (400, 500) into a distal portion of the lumen (102c) of the barrel (102b) such that the adapter assembly is operably positioned relative to the plunger assembly (110); and
engaging the adapter assembly within the distal portion of the lumen of the barrel.

10. The method according to claim 9, further including, prior to engaging the plug (106), moving a gripping portion (104a) of the trigger (104) of the handle assembly operably associated with the housing towards the stationary handle (102a) such that a stem portion (104b) of the trigger abuts a proximal end of the plunger assembly (110).

11. The method according to claim 9 or claim 10, wherein engaging the adapter assembly (400, 500) within the distal portion of the lumen (102c) of the barrel includes rotating a lock collar (120) relative to both the adapter assembly and the barrel.

## Patentansprüche

1. Auswechselbarer endoskopischer chirurgischer Klammerapplikator (10), umfassend:
eine Handgriffbaugruppe (100), umfassend:
ein Gehäuse (102) umfassend einen feststehenden Handgriff (102a) und ein Fass (102b), das an dem feststehenden Handgriff abgestützt ist, wobei das Fass ein proximales Ende, ein distales Ende und eine Längsachse umfasst, wobei das Fass ein Lumen (102c) definiert, das sich zwischen den proximalen und distalen Enden davon erstreckt, wobei das Lumen auf die Längsachse ausgerichtet ist und einen proximalen Abschnitt und einen distalen Abschnitt umfasst;
einen Auslöser (104), der betriebsmäßig mit dem Gehäuse (102) gekoppelt ist und relativ zu dem feststehenden Handgriff (102a) von einer nicht betätigten Position in eine betätigte Position beweglich ist;
eine Kolbenanordnung (110), die in dem proximalen Abschnitt des Lumens entfernbar positionierbar ist, wobei die Kolbenanordnung einen Kolben (112) mit einem Kopf (112a) und einem Schaft (112b), der sich distal von dem Kopf erstreckt und dazu konfiguriert ist, um sich betriebsmäßig mit dem Auslöser zu koppeln, so dass eine Bewegung des Auslösers von der nicht betätigten Position in die betätigte Position die Kolbenanordnung distal durch den proximalen Abschnitt des Lumens vorwärtsbewegt, umfasst; und
einen Stopfen (106), der mit dem proximalen Ende des Fasses (102b) entfernbar in Eingriff bringbar ist, um den proximalen Abschnitt des Lumens (102c) abzuriegeln, wodurch der Auslöser in betriebsmäßiger Beziehung zu der Kolbenanordnung (100) zurückgehalten wird, wobei der Klammerapplikator weiter umfasst:
eine Vielzahl von Adapteranordnungen (400, 500), von denen jede betriebsmäßig mit zumindest einem von einer Klammerkartuschenanordnung, einem Endeffektor oder einem chirurgischen Instrument verbunden ist, **dadurch gekennzeichnet, dass** zumindest eine der Adapteranordnungen umfasst:
eine Außenhülle (402);
einen Kragen (406), der verschiebbar in der Außenhülle angeordnet ist;
einen Schaft (404), der mit dem Kragen in Eingriff steht und sich distal von dem Kragen und der Außenhülle erstreckt; und
eine Kolbenanordnung (410), die verschiebbar in der Außenhülle angeordnet ist, wobei die Kolbenanordnung einen Stamm (412) und einen Kolben (414), der relativ zu dem Stamm verschiebbar ist, umfasst, wobei der Kolben einen Kopf (414a) und einen Schaft (414b), der sich distal von dem Kopf erstreckt, umfasst, wobei jede Adapteranordnung für einen lösbaren Empfang mit dem distalen Abschnitt des Lumens (102c) in betriebsmäßiger Beziehung relativ zu der Kolbenanordnung (110) der Handgriffbaugruppe (100) konfiguriert ist, so dass eine distale Bewegung des Kolbens (112) der Handgriffbaugruppe den Kolben (414) der zumindest einen Adapteranordnung distal vorantreibt, um die Klammerkartuschenanordnung, den Endeffektor oder das chirurgische Instrument, das mit der Adapteranordnung verbunden ist, zu betätigen.

2. Klammerapplikator nach Anspruch 1, wobei die Vielzahl von Adapteranordnungen (400, 500) eine Hublänge hat, die von einer Hublänge der verbleibenden Adapteranordnungen verschieden ist.

3. Klammerapplikator nach Anspruch 1 oder 2, wobei der Auslöser (104) einen Greifabschnitt (104a) und einen Stammabschnitt (104b) umfasst, wobei der Greifabschnitt relativ zu dem feststehenden Handgriff (102a) von einer nicht betätigten Position in die betätigte Position bewegbar ist, um den Stammabschnitt distal durch den proximalen Abschnitt des Lumens zu bewegen, um die Kolbenanordnung der Handgriffbaugruppe distal durch den proximalen Abschnitt des Lumens vorwärts zu bewegen.

4. Klammerapplikator nach Anspruch 1, 2 oder 3, wobei bei einer distalen Bewegung des Stammabschnitts (104b) durch den proximalen Abschnitt des Lumens (102c) der Stammabschnitt in Kontakt mit dem Kopf (112a) vorangetrieben wird, um hierdurch den Kolben (112) distal voranzutreiben, wobei die Kolbenanordnung (110) bevorzugt weiter einen Flansch (114), der verschiebbar um den Schaft (112b) herum angeordnet ist, und ein Rückstellelement (116), das um den Schaft herum angeordnet und zwischen dem Flansch und dem Kopf positioniert ist, umfasst, wobei noch bevorzugter der Flansch dazu konfiguriert ist, um an einer Schulter, die in dem proximalen Abschnitt des Lumens definiert ist, zur Anlage zu kommen, wenn die Kolbenanordnung darin positioniert ist, so dass das Rückstellelement bei einer distalen Bewegung des Kolbens durch den proximalen Abschnitt des Lumens komprimiert wird.

5. Klammerapplikator nach einem der vorstehenden Ansprüche, wobei die Handgriffbaugruppe (100) weiter einen Verriegelungskragen (120) umfasst, der drehbar mit den Fass (102b) gekoppelt ist und sich distal davon erstreckt, und wobei, mit einer der Adapteranordnungen in den distalen Abschnitt des Lumens (102c) eingesetzt, der Verriegelungskragen relativ zu dem Fass und der Adapteranordnung drehbar ist, um die Adapteranordnung lösbar in dem distalen Abschnitt des Lumens in Eingriff zu bringen.

6. Klammerapplikator nach einem der vorstehenden Ansprüche, wobei die Kolbenanordnung (410) der zumindest einen Adapteranordnung weiter ein Rückstellelement (416) umfasst, das um den Stamm (412) und den Schaft (414b) des Kolbens (414) herum angeordnet und zwischen dem Kragen (406) und dem Kopf (414a) des Kolbens positioniert ist, wobei die Kolbenanordnung derart konfiguriert ist, dass ein verschiebbares Einsetzen des Schafts des Kolbens in den Stamm das Rückstellelement komprimiert.

7. Klammerapplikator nach einem vorstehenden Anspruch, wobei der Stopfen (106) der Handgriffbaugruppe (100) für einen Gewindeeingriff in das proximale Ende des Fasses (102b) konfiguriert ist.

8. Klammerapplikator nach einem vorstehenden Anspruch, wobei das Gehäuse (102), der Auslöser (104), die Kolbenanordnung (110) und der Stopfen (106) als wiedersterilisierbare, wiederverwendbare Komponenten konfiguriert sind.

9. Verfahren zur Montage eines auswechselbaren endoskopischen chirurgischen Klammerapplikators (10) nach einem der vorstehenden Ansprüche, umfassend:
Einsetzen der Kolbenanordnung (110) der Handgriffbaugruppe (100) in einen proximalen Abschnitte des Lumens (102c), das in dem Fass (102b) des Gehäuses (102) definiert ist;
Ineingriffbringen des Stopfens (106) in dem proximalen Abschnitt des Lumens (102c) des Fasses, um den proximalen Abschnitt des Lumens des Fasses abzuriegeln und die Kolbenanordnung (110) darin zurückzuhalten;
Einsetzen einer Adapteranordnung (400, 500) in einen distalen Abschnitt des Lumens (102c) des Fasses (102b), so dass die Adapteranordnung betriebsmäßig relativ zu der Kolbenanordnung (100) positioniert ist; und
Ineingriffbringen der Adapteranordnung in dem distalen Abschnitt des Lumens des Fasses.

10. Verfahren nach Anspruch 9, weiter umfassend, vor dem Ineingriffbringen des Stopfens (106), ein Bewegen eines Greifabschnitts (104a) des Auslösers (104) der Handgriffbaugruppe, der betriebsmäßig mit dem Gehäuse verbunden ist, in Richtung des feststehenden Handgriffs (102a), so dass der Stammabschnitt (104b) des Auslösers an einem proximalen Ende der Kolbenanordnung (110) zur Anlage kommt.

11. Verfahren nach Anspruch 9 oder Anspruch 10, wobei das Ineingriffbringen der Adapteranordnung (400, 500) mit dem distalen Abschnitt des Lumens (102c) des Fasses ein Drehen eines Verriegelungskragens (120) relativ zu sowohl der Adapteranordnung als auch dem Fass umfasst.

## Revendications

1. Dispositif endoscopique réutilisable d'application d'agrafes chirurgicales (10), comprenant :
un ensemble poignée (100), incluant :
un logement (102) incluant une poignée immobile (102a) et un barillet (102b) supporté sur la poignée immobile, le barillet incluant une extrémité proximale, une extrémité distale et un axe longitudinal, le barillet définissant une lumière (102c) s'étendant entre les extrémités proximale et distale de ce dernier, la lumière étant alignée sur l'axe longitudinal et incluant une portion proximale et une portion distale ;
une gâchette (104) accouplée de manière opérationnelle au logement (102) et mobile par rapport à la poignée immobile (102a) d'une position non activée à une position activée ;
un ensemble piston (110) pouvant être positionné de façon amovible à l'intérieur de la portion proximale de la lumière, l'ensemble piston incluant un piston (112) ayant une tête (112a) et un arbre (112b) s'étendant de façon distale à partir de la tête et étant configuré pour s'accoupler de manière opérationnelle à la gâchette de sorte qu'un mouvement de la gâchette de la position non activée à la position activée avance l'ensemble piston de façon distale à travers la portion proximale de la lumière ; et
un bouchon (106) pouvant être mis en prise de façon amovible à l'intérieur de l'extrémité proximale du barillet (102b) pour fermer la portion proximale de la lumière (102c), conservant de ce fait la gâchette dans une relation opérationnelle par rapport à l'ensemble piston (100) ; le dispositif d'application d'agrafes comprenant en outre
une pluralité d'ensembles adaptateurs (400, 500), chacun d'eux pouvant être associé de manière opérationnelle à au moins l'un : d'un ensemble cartouche d'agrafes, d'un effecteur terminal, ou d'un outil chirurgical, **caractérisé en ce qu'**au moins un des ensembles adaptateurs inclut :
une coque extérieure (402) ;
un collier (406) disposé de manière coulissante à l'intérieur de la coque extérieure ;
un arbre (404) en prise avec le collier et s'étendant de façon distale à partir du collier et de la coque extérieure ; et
un ensemble piston (410) disposé de manière coulissante à l'intérieur de la coque extérieure, l'ensemble piston incluant une tige (412) et un piston (414) pouvant coulisser par rapport à la tige, le piston incluant une tête (414a) et un arbre (414b) s'étendant de façon distale à partir de la tête, dans lequel chaque ensemble adaptateur est configuré pour une réception amovible avec la portion distale de la lumière (102c) dans une relation opérationnelle par rapport à l'ensemble piston (110) de l'ensemble poignée (100) de sorte qu'un mouvement distal du piston (112) de l'ensemble poignée pousse le piston (414) de l'au moins un ensemble adaptateur de façon distale pour actionner l'ensemble cartouche d'agrafes, l'effecteur terminal, ou l'outil chirurgical associés à cet ensemble d'adaptateur.

2. Dispositif d'application d'agrafes selon la revendication 1, dans lequel la pluralité d'ensembles adaptateurs (400, 500) a une longueur de course qui est différente d'une longueur de course des ensembles adaptateurs restants.

3. Dispositif d'application d'agrafes selon la revendication 1 ou 2, dans lequel la gâchette (104) inclut une portion saisie (104a) et une portion tige (104b), la portion saisie étant mobile par rapport à la poignée immobile (102a) de la position non activée à la position activée pour déplacer la portion tige de façon distale à travers la portion proximale de la lumière pour avancer l'ensemble piston de l'ensemble poignée de façon distale à travers la portion proximale de la lumière.

4. Dispositif d'application d'agrafes selon la revendication 1, 2 ou 3, dans lequel lors d'un mouvement distal de la portion tige (104b) à travers la portion proximale de la lumière (102c), la portion tige est poussée en contact avec la tête (112a) pour pousser de ce fait le piston (112) de façon distale ; de préférence dans lequel l'ensemble piston (110) inclut en outre une bride (114) disposée de manière coulissante autour de l'arbre (112b) et un élément de sollicitation (116) disposé autour de l'arbre et positionné entre la bride et la tête ; toujours de préférence dans lequel la bride est configurée pour être contiguë à un épaulement défini à l'intérieur de la portion proximale de la lumière lorsque l'ensemble piston est positionné en son sein de sorte que l'élément de sollicitation est comprimé lors d'un mouvement distal du piston à travers la portion proximale de la lumière.

5. Dispositif d'application d'agrafes selon l'une quelconque des revendications précédentes, dans lequel l'ensemble poignée (100) inclut en outre un collier de verrouillage (120) couplé en rotation au barillet (102b) et s'étendant de façon distale à partir de là, et dans lequel, avec un des ensembles adaptateurs inséré dans la portion distale de la lumière (102c), le collier de verrouillage peut tourner par rapport au barillet et cet ensemble adaptateur pour mettre en prise de façon amovible cet ensemble adaptateur à l'intérieur de la portion distale de la lumière.

6. Dispositif d'application d'agrafes selon l'une quelconque des revendications précédentes, dans lequel l'ensemble piston (410) de l'au moins un ensemble adaptateur inclut en outre un élément de sollicitation (416) disposé autour de la tige (412) et l'arbre (414b) du piston (414) et positionné entre le collier (406) et la tête (414a) du piston, l'ensemble piston étant configuré de sorte que l'insertion pouvant coulisser de l'arbre du piston dans la tige comprime l'élément de sollicitation.

7. Dispositif d'application d'agrafes selon l'une quelconque des revendications précédentes, dans lequel le bouchon (106) de l'ensemble poignée (100) est configuré pour une mise en prise filetée à l'intérieur de l'extrémité proximale du barillet (102b).

8. Dispositif d'application d'agrafes selon l'une quelconque des revendications précédentes, dans lequel le logement (102), la gâchette (104), l'ensemble piston (110) et le bouchon (106) sont configurés comme des composants réutilisables, pouvant être stérilisés plusieurs fois.

9. Procédé d'assemblage d'un dispositif endoscopique réutilisable d'application d'agrafes chirurgicales (10) selon l'une quelconque des revendications précédentes, comprenant :
l'insertion de l'ensemble piston (110) de l'ensemble poignée (100) dans une portion proximale de la lumière (102c) définie à l'intérieur du barillet (102b) du logement (102);
la mise en prise du bouchon (106) à l'intérieur de la portion proximale de la lumière (102c) du barillet pour fermer la portion proximale de la lumière du barillet et retenir l'ensemble piston (110) en son sein ;
l'insertion d'un ensemble adaptateur (400, 500) dans une portion distale de la lumière (102c) du barillet (102b) de sorte que l'ensemble adaptateur est positionné de manière opérationnelle par rapport à l'ensemble piston (110) ; et
la mise en prise de l'ensemble adaptateur à l'intérieur de la portion distale de la lumière du barillet.

10. Procédé selon la revendication 9, incluant en outre, avant de mettre en prise le bouchon (106), le déplacement d'une portion saisie (104a) de la gâchette (104) de l'ensemble poignée associé de manière opérationnelle au logement vers la poignée immobile (102a) de sorte qu'une portion tige (104b) de la gâchette est contiguë à une extrémité proximale de l'ensemble piston (110).

11. Procédé selon la revendication 9 ou la revendication 10, dans lequel la mise en prise de l'ensemble adaptateur (400, 500) à l'intérieur de la portion distale de la lumière (102c) du barillet inclut la rotation d'un collier de verrouillage (120) par rapport tant à l'ensemble adaptateur qu'au barillet.
